Europäisches Patentamt

European Patent Office

Office européen des brevets

(11) Veröffentlichungsnummer : **0 085 957**
**B1**

(12) **EUROPÄISCHE PATENTSCHRIFT**

(45) Veröffentlichungstag der Patentschrift :
**30.12.86**

(21) Anmeldenummer : **83101031.9**

(22) Anmeldetag : **03.02.83**

(51) Int. Cl.⁴ : **A 61 M   5/16**

(54) **Vorrichtung zur Entnahme von Flüssigkeiten aus insbesondere steril verschlossenen Gefässen.**

(30) Priorität : **05.02.82 DE 3203954**

(43) Veröffentlichungstag der Anmeldung :
**17.08.83 Patentblatt 83/33**

(45) Bekanntmachung des Hinweises auf die Patenterteilung : **30.12.86 Patentblatt 86/52**

(84) Benannte Vertragsstaaten :
**DE FR GB IT NL**

(56) Entgegenhaltungen :
**DE-A- 2 834 588**
**DE-B- 1 138 512**
**DE-B- 2 022 107**
**DE-B- 2 116 442**
**FR-A- 1 105 576**

(73) Patentinhaber : **Fresenius AG**
**Gluckensteinweg 5**
**D-6380 Bad Homburg (DE)**

(72) Erfinder : **Mathieu, Bernd**
**Am unteren Galgenberg 19**
**D-6683 Spiesen (DE)**
Erfinder : **Weber, Wolfram**
**Albert-Schweitzer-Strasse 33**
**D-6683 Spiesen (DE)**

(74) Vertreter : **Luderschmidt, Wolfgang, Dr. Dipl-Chem.**
**Görtz, Dr. Fuchs, Dr. Luderschmidt Patentanwälte**
**Sonnenberger Strasse 100 Postfach 26 26**
**D-6200 Wiesbaden (DE)**

EP 0 085 957 B1

**Beschreibung**

Die Erfindung betrifft eine Vorrichtung zur Entnahme von Flüssigkeiten aus insbesondere steril verschlossenen Gefäßen nach dem Oberbegriff des Anspruchs 1.

Derartige Vorrichtungen sind aus dem medizinischen Bereich z. B. aus der DE-B-2 116 442 und der DE-A-2 843 588 als Universalinfusionsgeräte bekannt, deren Ausbildung es erlaubt, die Flüssigkeit dem Gefäß unter allen in der Praxis vorkommenden Bedingungen zu entnehmen. Bei diesen Geräten wird das durch die Entnahme verminderte Flüssigkeitsvolumen in dem Gefäß durch die Zufuhr von Luft aus der Umgebung ersetzt. Um einen Eintritt von in der Umgebungsluft enthaltenen Keimen in das Gefäßinnere zu vermeiden, ist ein Luftfilter vorgesehen, durch den hindurch die zum Ersatz des Flüssigkeitsvolumens nötige Luft in das Infusionsgerät und von dort durch einen Luftkanal in das Gefäßinnere steril gefiltert gelangt. Dieses Luftfilter muß außerdem die Funktion einer Flüssigkeitsdichtung ausüben, da beispielsweise bei Druckinfusionen, bei denen im Gefäßinneren ein Überdruck herrscht, Flüssigkeit in den Luftkanal bis zum Luftfilter dringt, der aus diesem Grunde aus hydrophobem Material besteht und somit einen Flüssigkeitsaustritt aus dem Infusionsgerät verhindert.

Werden bei der drucklosen Infusion Infusionsgefäße verwendet, die das entnommene Flüssigkeitsvolumen nicht durch eigene Verformung ersetzen, sondern durch Belüftung des Gefäßinneren, ist ein gewisser Unterdruck im Gefäßinneren zur Bildung der ersten Luftblase auf dem Luftfilter und damit zur Vergrößerung der Oberfläche der Flüssigkeit nötig, wenn das Gefäß belüftet werden soll. Andererseits kollabieren sehr viele, dünnwandige Infusionsgefäße schon bei sehr niedrigen Unterdrücken, so daß die Belüftung des Gefäßes unterhalb dieses Unterdrucks beginnen muß. Da die Größe des Unterdrucks von der Porengröße und dem Material des Luftfilters abhängt, könnte dieses Problem durch Verwendung dementsprechend ausgebildeter Luftfilter gelöst werden.

Hierbei ist jedoch nachteilig, daß derartige Luftfilter nur bis weniger als 0,5 bar Druck wasserdicht sind, was für Druckinfusionen nicht ausreicht. Daher werden im allgemeinen bei Universalinfusionsgeräten Luftfilter verwendet, die auch bei den bei Druckinfusionen auftretenden Überdrücken wasserdicht sind. Bei drucklosen Infusionen benötigen diese Luftfilter jedoch einen höheren Unterdruck zur Bildung der ersten Luftblase. Andererseits kollabieren jedoch die meisten, sehr dünnwandigen bei drucklosen Infusionen verwendeten Infusionsgefäße schon bei wesentlich geringerem Unterdruck als dem, der zur Bildung der ersten Luftblase nötig ist. Da nun Universalinfusionsgeräte sowohl für die drucklose Infusion als auch für die Druckinfusion verwendet werden, besteht bei diesen Geräten der Nachteil, daß sie entweder bei Druckinfusionen nicht ausreichend dicht sind, oder die verwendeten dünnwandigen Infusionsgefäße nicht rechtzeitig vor dem kollabieren belüften.

Es ist zwar versucht worden, dieses Problem durch Einbau eines Rückschlagventiles mit Kugel (FR-A-1 105 571) oder Gummisegel zu lösen, das ein Zurücklaufen von Infusionsflüssigkeit an den Luftfilter verhindern soll. Dieses Ventil besitzt jedoch den Nachteil, daß es ebenfalls einen gewissen Druck zur Öffnung benötigt und zudem in gewissem Umfange lageabhängig ist. Außerdem kann es bei Druckinfusionen oder längerem Stehenlassen des Gerätes ohne Benutzung zum Verkleben des Ventils kommen.

Es ist daher Aufgabe der Erfindung, eine Vorrichtung der im Oberbegriff des Anspruchs 1 umrissenen Gattung zu schaffen, die sowohl bei der Druckentnahme von Flüssigkeiten aus verschlossenen Gefäßen dicht ist, als auch bei einer drucklosen Entnahme von Flüssigkeiten eine sichere Belüftung des Gefäßes ermöglicht.

Diese Aufgabe wird durch die kennzeichnenden Merkmale des Anspruchs 1 gelöst.

Dadurch wird erreicht, daß auch bei bis zum Luftfilter vordringender Flüssigkeit eine gewisse Luftmenge, die sich in der Speicherkammer sammelt, in der erfindungsgemäßen Vorrichtung verbleibt. Es hat sich nämlich überraschenderweise gezeigt, daß der notwendige Unterdruck zu Beginn der Belüftung drastisch absenkbar ist, falls bereits Luft auf der Innenseite des Luftfilters vorhanden ist. Da jedoch auch bei der drucklosen Infusion Flüssigkeit bis zum Luftfilter vordringen kann, wird diese in der Luftleitung der Vorrichtung befindliche nötige Luft durch den Luftfilter hindurch verdrängt. Mit der erfindungsgemäßen Vorrichtung ist es möglich, ein Luftpolster auch bei in die Luftleitung eindringender Flüssigkeit in der Speicherkammer aufzunehmen, aus der sich die Luft bei sich in der Vorrichtung aufbauendem Unterdruck ausdehnt und sich entweder als wandernde Luftfront oder unter Bildung einzelner Blasen an dem Luftfilter anlegt.

Somit ist es möglich, den Luftfilter aus einem Material auszubilden, das auch bei einer Entnahme von Flüssigkeiten unter Druck die Dichtheit der erfindungsgemäßen Vorrichtung gewährleistet und andererseits der vor dem Kollabieren des die Flüssigkeit enthaltenden Gefäßes herrschende Unterdruck zur sicheren Belüftung des Gefäßes ausreicht. Ein weiterer Vorteil besteht darin, daß sowohl der Luftfilter als auch die Speicherkammer an jeder beliebigen geeigneten Stelle der erfindungsgemäßen Vorrichtung anordenbar ist, so daß eine konstruktiv und kostenmäßig günstige Ausbildung der erfindungsgemäßen Vorrichtung möglich ist.

Die Unteransprüche 2 bis 11 haben vorteilhafte Weiterbildungen der Erfingung zum Inhalt.

Weitere Einzelheiten, Merkmale und Vorteile der Erfindung ergeben sich aus der folgenden Beschreibung von fünf Ausführungsformen anhand der Zeichnung.

Es zeigt

Figur 1 einen Vertikalschnitt durch eine erste Ausführungsform einer erfindungsgemäßen Vorrichtung,

Figur 2 eine Fig. 1 entsprechende Darstellung einer zweiten Ausführungsform der erfindungsgemäßen Vorrichtung,

Figur 3 eine Fig. 1 und 2 entsprechende Darstellung einer dritten Ausführungsform der erfindungsgemäßen Vorrichtung,

Figur 4 eine Fig. 1 bis 3 entsprechende Darstellung einer vierten Ausführungsform der erfindungsgemäßen Vorrichtung,

Figur 5 eine den Fig. 1 bis 4 entsprechende Darstellung einer besonders bevorzugten Ausführungsform der erfindungsgemäßen Vorrichtung als Universalinfusionsgerät,

Figur 6 bis 8 eine Darstellung einer Einzelheit aus Fig. 5 unter verschiedenen Druckbedingungen,

Figur 9 eine Fig. 5 entsprechende Darstellung einer weiteren besonders bevorzugten Ausführungsform der erfindungsgemäßen Vorrichtung als Universalinfusionsgerät,

Figur 10 eine vergrößerte Seitenansicht der Ausführungsform gemäß Fig. 9 aus Richtung des Pfeiles X in Fig. 9,

Figur 11 eine vergrößerte Schnittansicht der Vorrichtung gemäß Fig. 9 und 10 entlang der Linie XI-XI in Fig. 10.

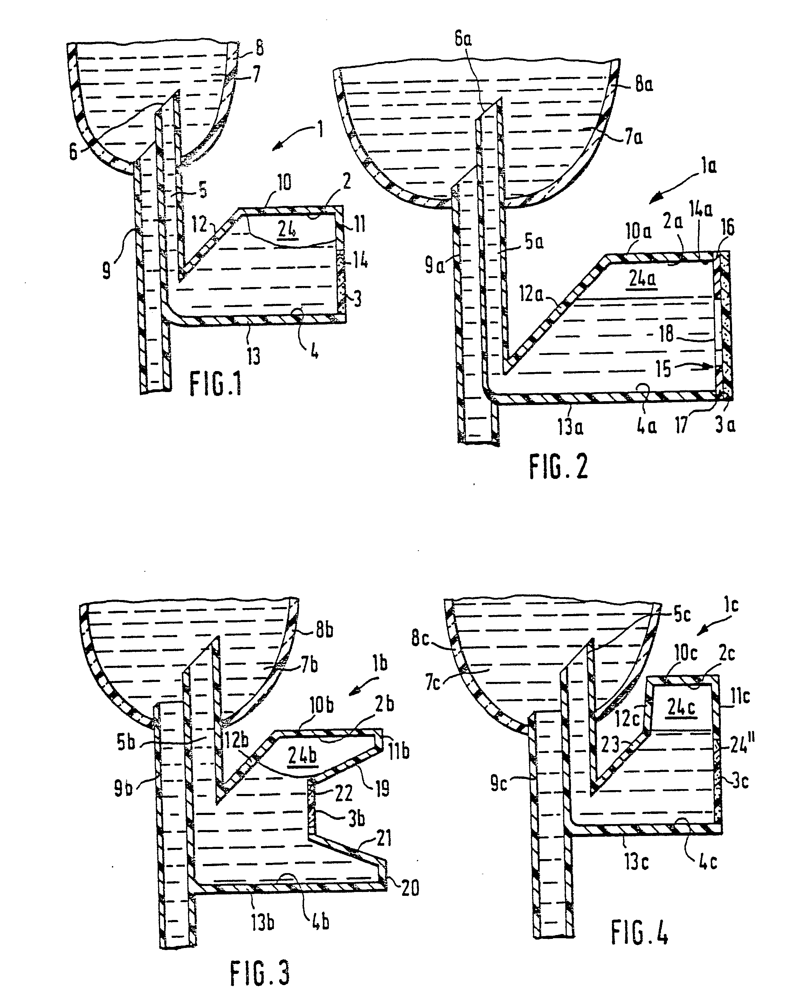

In den Fig. 1 bis 4 sind vier Ausführungsformen einer erfindungsgemäßen Vorrichtung dargestellt, die sich im wesentlichen durch die unterschiedlichen Ausbildungen einer Speicherkammer unterscheiden. Gemäß Fig. 1 weist eine Vorrichtung 1 eine Speicherkammer 2 auf, die an den oberen Bereich einer an einen Luftfilter 3 angrenzenden Kammer 4 angeordnet ist. Die Kammer 4 steht mit einer Luftleitung 5 in Verbindung, deren in der Zeichnung oberes Ende 6 in einem eine Flüssigkeit 7 enthaltenden geschlossenen Gefäß 8 endet. Unmittelbar neben der Luftleitung, im unteren Bereich des Gefäßes 8 mündet eine Flüssigkeitsleitung 9 zur Entnahme der Flüssigkeit 7 aus dem Gefäß 8. Die Speicherkammer 2 weist eine Deckwand 10 und zwei Seitenwände 11 und 12 auf, wobei die Seitenwand 11 im rechten Winkel an die Deckwand 10 anschließt und mit einer Bodenwand 13 der Kammer 4 eine Ausnehmung 14 begrenzt, in die der als hydrophobe Membran ausgebildete Luftfilter 3 eingesetzt ist. Die Seitenwand 12 schließt sich im stumpfen Winkel an die Deckwand 10 an und endet am Eintritt der Luftleitung 5 in die Kammer 4. Die Deckwand 10, die Bodenwand 13 und die Seitenwände 11 und 12 sind luft- und wasserdicht ausgebildet, während der Luftfilter 3 zwar wasserdicht, jedoch luftdurchlässig ist. Die Seitenwand 11 kann durch eine luftdichtmachende Abdichtung eines im Einbauzustand des Luftfilters 3 oberen Teils des Luftfilters 3 mit Hilfe von Beschichtungsmitteln gebildet werden. Dies bringt den Vorteil mit sich, daß lediglich ein entsprechend modifiziertes Luftfilter 3 ohne sonstige Änderungen an der Vorrichtung 1 eingebaut werden muß.

In Fig. 2 ist eine zweite Ausführungsform der erfindungsgemäßen Vorrichtung dargestellt, in der gleiche Teile mit gleichen Bezugszeichen, jedoch mit dem Index a bezeichnet sind. Demgemäß weist die Vorrichtung 1a die Speicherkammer 2a auf, die sich in der gemäß Fig. 1 entsprechenden Weise an die an den Luftfilter 3a angrenzende Kammer 4a anschließt. Die Luftleitung 5a mündet mit ihrem Ende 6a im Behälter 8a, während die Flüssigkeitsleitung 9a im unteren Bereich des Gefäßes 8a mündet.

Im Unterschied zur Ausführungsform gemäß Fig. 1 ist die Ausnehmung 14a, in die das Luftfilter 3a eingesetzt ist, von der Deckwand 10a und der Bodenwand 13a begrenzt. Auf das Luftfilter 3a ist von innen ein Ring 15 aufgesetzt, der mit seinen Randbereichen 16 und 17 an der Abdeckwand 10a bzw. der Bodenwand 13a festgelegt ist. Die Randbereiche 16 bzw. 17 decken die entsprechenden Bereiche des Luftfilters 3a luftdicht ab, so daß nur über eine einer Ausnehmung 18 des Ringes 15 entsprechende Fläche des Luftfilters 3a Luft aus der Kammer 4a entweichen kann. Diese Ausführungsform bietet sich an, falls Ringe 15 einer der Ausnehmung 14a entsprechenden Größe erhältlich sind, so daß auf einfache und kostengünstige Weise eine Unterteilung des Luftfilters 3a in luftdichte und luftdurchlässige Bereiche möglich ist, ohne daß weitere bauliche Änderungen an der Vorrichtung 1a nötig sind.

In Fig. 3 ist eine dritte Ausführungsform der erfindungsgemäßen Vorrichtung dargestellt, in der entsprechende Teile mit gleichen Bezugszeichen jedoch mit dem Index b versehen sind. Die Vorrichtung 1b weist demgemäß eine ringförmige Speicherkammer 2b auf, die sich an den oberen Bereich der an das Luftfilter 3b angrenzenden Kammer 4b anschließt. Weiterhin weist die Vorrichtung 1b eine Luftleitung 5b und eine Flüssigkeitsleitung 9b auf, die in dem Behälter 8b zur Aufnahme der Flüssigkeit 7b münden. Die Speicherkammer 2b weist eine Deckwand 10b und zwei Seitenwände 11b und 12b auf, wobei die Seitenwand 12b im stumpfen Winkel an die Deckwand 10b anschließt und am Eintritt der Luftleitung 5b endet. Die Seitenwand 11b schließt sich im rechten Winkel an die Deckwand 10b an und ist mit einer Wand 19 verbunden, die sich von der Seitenwand 11b aus im stumpfen Winkel zu dieser nach innen erstreckt. An die Bodenwand 4b schließt sich eine aufrechte Seitenwand 20 an, die mit einer sich nach innen erstreckenden und im stumpfen Winkel zur Seitenwand 20 angeordneten Wand 21 verbunden ist. Die Endbereiche der Wände 19 und 21 begrenzen eine Ausnehmung 22, in die das Luftfilter 3b eingesetzt ist.

Die die Speicherkammer begrenzenden Wände 10b, 11b, 12b und 19 und die Wände 4b, 20 und 21 sind luft- und wasserundurchlässig, während das Luftfilter 3b zwar luftdurchlässig, jedoch wasserundurchlässig ist.

Diese dritte Ausführungsform bringt den Vorteil mit sich, daß die Vorrichtung 1b weitgehend lageunabhängig ist, da die Speicherkammer 2b einen Ringraum bildet, aus der eine einmal eingeschlossene Luftblase auch bei Lageänderungen der Vorrichtung 1b nicht entweichen und damit nicht vom Luftfilter 3b abgesaugt werden kann.

Gemäß Fig. 4 ist eine vierte Ausführungsform der erfindungsgemäßen Vorrichtung dargestellt, in der gleiche Teile mit gleichen Bezugszeichen, jedoch mit dem Index c versehen sind.

Demgemäß weist die Vorrichtung 1c die Speicherkammer 2c auf, die sich an den oberen Bereich der an das Luftfilter 13c angrenzenden Kammer 4c anschließt. Die Luftleitung 5c und die Flüssigkeitsleitung 9c münden in der oben beschriebenen Weise in dem die Flüssigkeit 7c enthaltenen Gefäß 8c. Die Speicherkammer 2c ist durch die Abdeckwand 10c und die Seitenwände 11c und 12c begrenzt, wobei bei dieser Ausführungsform die Seitenwand 12c im rechten Winkel auf der Deckwand 10c steht und sich an eine in der Zeichnung schräg nach unten verlaufende Wand 23 anschließt, die ihrerseits mit der Luftleitung 5c verbunden ist.

Die sich im rechten Winkel an die Deckwand 10 anschliessende Seitenwand 11c begrenzt mit der Bodenwand 13c der Kammer 4c eine Ausnehmung 24″, in die das Luftfilter 3c eingesetzt ist. Durch diese Anordnung entsteht eine kuppelförmige Ausbildung der Speicherkammer 2c, die wasser- und luftundurchlässig ist, während auch bei dieser Ausführungsform das Luftfilter wasserdicht, jedoch luftdurchlässig ist. Diese Ausführungsform bietet sich an, wenn die Wand 10c beispielsweise als runder Deckel auf der kuppelförmig ausgebildeten, zylindrischen Speicherkammer 2c nachträglich beispielsweise durch Aufschweißen oder Aufkleben angeordnet werden kann.

Allen vier Ausführungsformen ist gemeinsam, daß bei in die Luftleitung 5 bzw. 5a, 5b, 5c eindringender Flüssigkeit 7 bzw. 7a, 7b, 7c die in der Luftleitung 5 bzw. 5a, 5b, 5c befindliche Luft zum Teil in die Kammer 4 bzw. 4a, 4b, 4c verdrängt wird und von dieser aus durch das luftdurchlässige Filter 3 bzw. 3a, 3b, 3c aus der Vorrichtung 1 bzw. 1a, 1b, 1c, entweichen kann. Der Teil der Luft jedoch, der in die Speicherkammer 2 bzw. 2a, 2b, 2c verdrängt wird, verbleibt als Luftpolster 24 bzw. 24a, 24b, 24c in der Vorrichtung 1 bzw. 1a, 1b, 1c, da die Speicherkammer 2 bzw. 2a, 2b, 2c luftundurchlässig ist. Da sich das Luftpolster 24 bzw. 24a, 24b, 24c aufgrund der Anordnung der Speicherkammer 2 bzw. 2a, 2b, 2c in der Nachbarschaft des Luftfilters 3 bzw. 3a, 3b, 3c befindet, ist bei im Gefäß 8 bzw. 8a, 8b, 8c entstehenden Unterdruck ein Ausdehnen des Luftpolsters 24 bzw. 24a, 24b, 24c in Richtung auf das Luftfilter 3 bzw. 3a, 3b, 3c und damit die Bildung einer ersten zur Belüftung des Gefäßes 8 bzw. 8a, 8b, 8c nötigen Luftblase auf dem Luftfilter 3 bzw. 3a, 3b, 3c möglich.

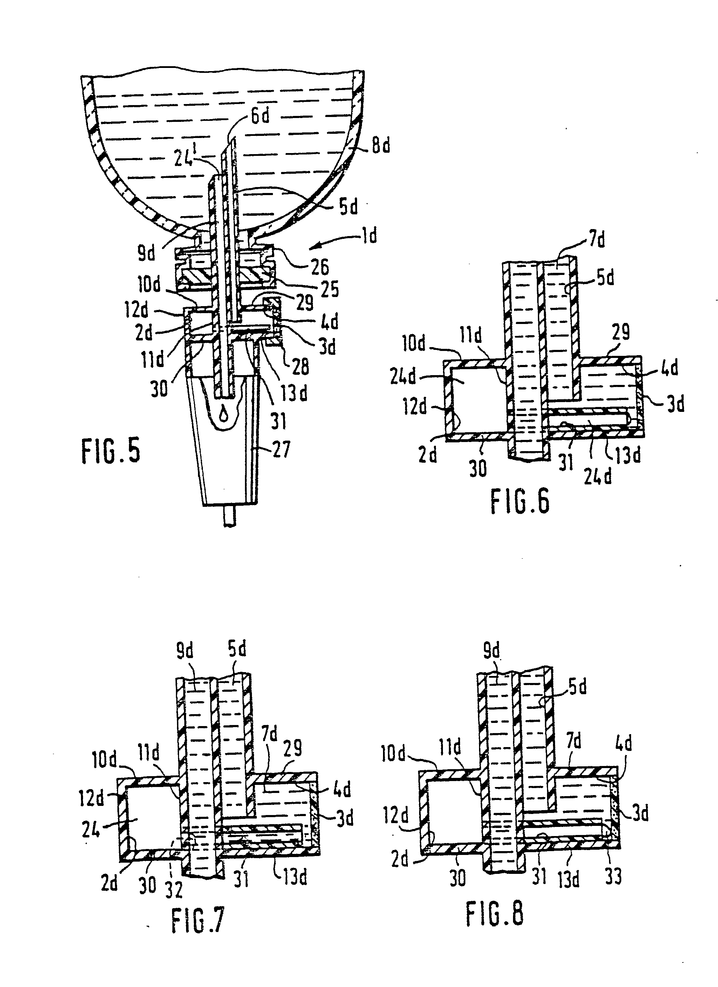

Gemäß Fig. 5 ist eine fünfte Ausführungsform der erfindungsgemäßen Vorrichtung dargestellt, bei der gleiche Teile mit gleichen Bezugszeichen, jedoch mit dem Index d bezeichnet sind. Diese Ausführungsform eignet sich insbesondere zur Anwendung als Universalinfusionsgerät, das bei drucklosen Infusionen wie auch bei Druckinfusionen Verwendung findet.

Die Luftleitung 5d und die Flüssigkeitsleitung 9d der Vorrichtung 1d sind einstückig aneinander angeformt und weisen spitz zulaufende Endbereiche 6d bzw. 24′ auf. Diese nach Art einer Injektionsnadel ausgebildeten Leitungen durchstoßen ein Dichtteil 25, das in einem bodenseitigen Halteteil 26 des als Infusionsflasche ausgebildeten Gefäßes 8d gelagert ist und dieses abdichtet. Die Flüssigkeitsleitung endet im unteren Bereich des Gefäßes 8d und leitet die entnommene Flüssigkeit in eine Tropfkammer 27, die an der Vorrichtung 1d angebracht ist. Die Luftleitung 5d mündet oberhalb der Flüssigkeitsleitung 9d im Gefäß 8d und endet in der an das Luftfilter 3d angrenzenden Kammer 4d. Das als hydrophobe Membran ausgebildete Luftfilter 3d verschließt die Kammer 4d gegen die Umgebung und ist mittels eines Sicherungsringes 28 an der Bodenwand 13d und einer Deckwand 29 der Kammer 4d festgelegt.

Die Speicherkammer 2d ist bei dieser Ausführungsform an die Flüssigkeitsleitung 9d angrenzend angeordnet und ist durch die Deckwand 10d, die Seitenwände 11d und 12d und eine Bodenwand 30 begrenzt. Im Beispielsfalle bildet die Seitenwand 11d zugleich eine Außenwand der Flüssigkeitsleitung 9d, so daß die Speicherkammer 2d ein integrales Bauteil der Vorrichtung 1d mit einem Volumen von wenigstens annähernd 50 µl bis maximal annähernd 1 000 µl, vorzugsweise annähernd 100 bis 200 µl, bildet. Die Speicherkammer 2d ist mit der an die Innenseite des Luftfilters 3d angrenzenden Kammer 4d mittels eines Luftkanals 31 verbunden, der einen Innendurchmesser von wenigstens annähernd 0,3 bis maximal annähernd 3 mm, vorzugsweise zwischen 1 und 2 mm aufweist. Insbesondere im Falle eines aufrecht angeordneten Luftfilters 3d endet der im Beispielsfalle auf der Bodenwand 13d liegende Luftkanal 31 im geringen Abstand von wenigstens annähernd 0,1 bis maximal 2 mm, vorzugsweise annähernd 0,3 bis 0,8 mm vor dem Luftfilter 3d.

Bei einem ebenfalls denkbaren Einbau des Luftfilters 3d, der, wie auch die Luftfilter 3a, 3b, 3c, als hydrophobe Membran mit einer Porengröße von wenigstens annähernd 0,1 bis 2 µm, vorzugsweise von annähernd 0,5 bis 1,2 µm ausgebildet ist, beispielsweise in der Deckwand 29 in horizontaler Lage müssen die letztgenannten Abstände des Luftkanals 31 von der Innenseite des Luftfilters 3d nicht unbedingt eingehalten werden.

Die Wirkungsweise der Vorrichtung 1d in der Ausführungsform gemäß Fig. 5 bei der Anwendung als Universalinfusionsgerät wird im folgenden in Verbindung mit den Fig. 6 bis 8 erläutert, die einen vergrößerten Ausschnitt eines Teiles der Vorrichtung 1d bei verschiedenen Druckbedingungen darstellen. Die Darstellung gemäß Fig. 6 zeigt einen Zustand, wie er beispielsweise nach dem Aufhängen des als Infusionsflasche ausgebildeten Gefäßes 8d entsteht, wenn beim Aufhängen durch Druckausübung auf die Infusionsflasche Flüssigkeit über die Luftleitung bis zum Luftfilter 3d vorgedrungen ist. In diesem Zustand ist die auf dem Luftfilter 3d anliegende Flüssigkeitsoberfläche eben und energetisch gesehen auf einem Minimum. Aus diesem Grunde dehnt sich das in der Speicherkammer 2d enthaltene Luftpolster 24d bis an das Ende des Luftkanals 31 aus und wölbt sich leicht in die Flüssigkeit hinein, ohne jedoch dabei mit der Innenseite der Membran 3d in Berührung zu kommen.

Die Darstellung gemäß Fig. 7 entspricht einem Zustand, der bei Druckinfusionen entsteht. Dabei steht die in dem Gefäß 8d enthaltene Flüssigkeit 7d unter Druck und wird über die Luftleitung 5d in die Kammer 4d und von dieser aus in den Luftkanal 31 gedrückt, wobei das Luftpolster 24d solange in dem Speicherraum 2d komprimiert wird, bis es der Flüssigkeitssäule das Gleichgewicht hält. In diesem Falle reicht die Flüssigkeit 7d bis in die Nachbarschaft des Endbereiches 32 des Luftkanals 31, der in die Speicherkammer 2d mündet.

Gemäß Fig. 8 ist ein Zustand dargestellt, der ausgehend von demjenigen gemäß Fig. 6 dann entsteht, wenn Flüssigkeit aus dem Behälter 8d über die Flüssigkeitsleitung 9d entnommen worden ist. In diesem Fall entsteht in dem Gefäß 8d ein leichter Unterdruck, der eine Ausdehnung des Luftpolsters 24d aus dem Luftkanal 31 heraus bewirkt, so daß eine dabei entstehende Luftblase 33 an die Innenseite des Luftfilters 3d zu liegen kommt. Aufgrund des geringen Abstandes des Luftkanals 31 von der Innenseite des Luftfilters 3d reicht ein schon sehr geringer Unterdruck im Gefäß 8d zur Bildung der die Innenseite des Luftfilters 3d berührenden Luftblase 33 aus. Damit ist es möglich, das Luftfilter 3d aus einem Material auszubilden, das bei einer Druckinfusion, wie sie in Fig. 7 dargestellt ist, die Dichtheit des Universalinfusionsgerätes zu gewährleisten, und trotzdem die sichere Belüftung des Gefäßes 8d sicherstellt, da schon nach wenigen Millilitern Flüssigkeitsentnahme herrschender geringer Unterdruck dazu ausreicht, die erste Luftblase auf der Innenseite des Luftfilters 3d zu bilden, wodurch wiederum ein kollabieren des Gefäßes 8d vermeidbar ist.

Bei Überdruck, beispielsweise bei Druckinfusionen, wird die Luft in der Kammer 2d komprimiert und die Kammer 2d teilweise mit Flüssigkeit 7d gefüllt. Bei Entfernen des Überdrucks dehnt sich die Luft erneut aus und fördert die Flüssigkeit 7d aus der Kammer 2d heraus. Zu diesem Zweck ist es nützlich, den Eingang bzw. Ausgang der Kammer 2d, also den Luftkanal 31, am tiefsten Punkt der Kammer 2d anzuordnen. Aufgrund der Oberflächenspannung der Flüssigkeit 7d und der nur bedingten Benetzbarkeit der Kunststoffmassen ist diese Ausführung relativ lageunabhängig wirksam, da die Flüssigkeit 7d als Tropfen in der Kammer 2d liegt und über den Luftkanal 31 immer Verbindung mit dem Inneren der Kammer 4d behält. Dadurch wird die Luft komprimiert und immer zuerst die Flüssigkeit 7d aus der Kammer 2d herausgedrückt, bevor Luft aus der Kammer 2d entweichen kann.

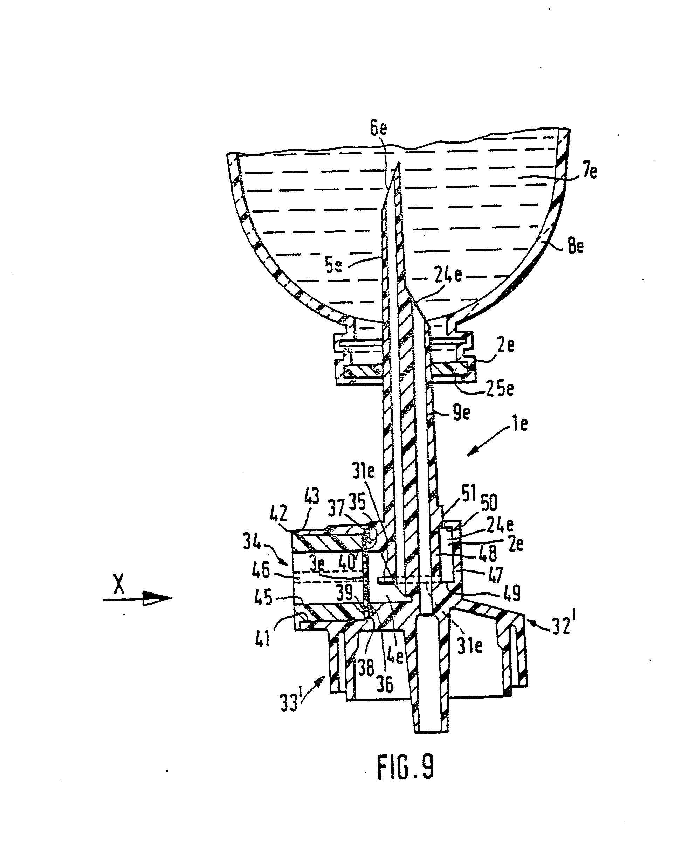

Gemäß Fig. 9 ist eine sechste Ausführungsform der erfindungsgemäßen Vorrichtung dargestellt, bei der gleiche Teile mit gleichen Bezugszeichen, jedoch mit dem Index e bezeichnet sind. Diese Ausführungsform eignet sich ebenfalls insbesondere zur Anwendung als Universalinfusionsgerät.

In einer der Ausführungsform gemäß Fig. 5 entsprechenden Weise weist die Vorrichtung 1e die Luftleitung 5e und die Flüssigkeitsleitung 9e auf, die einstückig aneinander angeformt sind und spitz zulaufende Endbereiche 6e bzw. 24e aufweisen. Diese nach Art einer Injektionsnadel ausgebildeten Leitungen durchstoßen ein Dichtteil 25e, das in einem bodenseitigen Halteteil 26e des als Infusionsflasche ausgebildeten Gefäßes 8e gelagert ist und dieses abdichtet. Die Flüssigkeitsleitung endet im unteren Bereich des Gefäßes 8e und leitet die entnommene Flüssigkeit in eine nicht näher dargestellte Tropfkammer, die an zwei Halteelementen 32' und 33' an der Vorrichtung 1e anbringbar ist. Die Luftleitung 5e mündet oberhalb der Flüssigkeitsleitung 9e im Gefäß 8e und endet in der an das Luftfilter 3e angrenzenden Kammer 4e. Das als hydrophe Membran ausgebildete Luftfilter 3e verschließt die Kammer 4e gegen die Umgebung wobei es in der Richtung von der Kammer 4e in die Umgebung als Flüssigkeitsdichtung wirkt, die in die Kammer 4e vorgedrungene Flüssigkeit zurückhält und in umgekehrter Richtung in die Kammer angesaugte Luft aus der Umgebung bakterienfrei filtert. Im Beispielsfalle ist das Luftfilter 3e mittels eines als Stopfen ausgebildeten Halteteils 34 gegen zwei kegelförmig angefaste Kantenbereiche 35 bzw. 36 festgelegt, wobei die scharfen Kantenbereiche 35 u. 36 eine ringförmige Dichtlippe zur luftdichten Abdichtung des Luftfilters 3e bilden. Durch die kegelförmige Anfasung zweier Gehäuseschultern 37 und 38 entstehen zwei kegelförmige Spaltbereiche 39 und 40, in die sich das Luftfilter 3e unter dem Druck, den das Halteteil 34 auf das Luftfilter 3e ausübt, etwas ausdehnen kann wodurch ein Herausrutschen des Luftfilters 3e aus der eine Klemmverbindung darstellenden Halterung des Luftfilters 3e an den Gehäuseschultern 37 und 38 verhindert wird.

Das Halteteil 34 ist als zylinderförmiger Stopfen mit einem Kreisringquerschnitt ausgebildet. Das Halteteil 34 ist in einem zylinderförmigen Raum 41 angeordnet, dessen Innendurchmesser und Länge dem Außendurchmesser und der Länge des Halteteiles entspricht. Zur Festlegung des Luftfilters 3e in der Vorrichtung 1e wird das Halteteil 34 in dem Raum 41 derart angeordnet, daß es das Luftfilter 3e gegen die Kanten 36 bzw. 37 drückt. Zur Festlegung des Halteteils 34 in dem Raum 41 weist das Halteteil 34 eine sich radial nach außen erstreckende ringförmige Schulter 42 auf, mittels derer das Halteteil 34 an einer nach außen weisenden ringförmigen Stirnfläche 43 des Raumes 41 beispielsweise durch Verkleben festgelegt ist. Das Halteteil 34 weist eine Ausnehmung 45 auf, die über die gesamte Länge des Halteteils 34 verläuft und in der ein kreuzförmiges Einlageteil 46 bleibend angeordnet ist, das eine Schutzeinrichtung für das Luftfilter 3e gegen Beschädigung bildet. In die Ausnehmung 45 des Halteteils 34 kann außerdem ein aus luftdichtem Material bestehender Stopfen eingebracht werden, falls es nötig oder erwünscht ist, das Luftfilter 3e ganz oder teilweise luftundurchlässig zu machen.

Die Speicherkammer 2e ist im Beispielsfalle als Ringraum ausgebildet, der sich über einen Winkel von 180° der Vorrichtung 1e erstreckt. Die Speicherkammer 2d wird von Seitenwänden 47 und 48 und einer Bodenwand 49 begrenzt und weist eine in der Zeichnung nach oben weisende Öffnung 50 auf, die mittels eines nachträglich aufsetzbaren Deckels 51 verschließbar ist. Der Deckel 51 weist im Beispielsfalle einen L-förmigen Querschnitt auf und kann beispielsweise durch Verkleben oder Verschweißen befestigt werden. Der Luftkanal 31e tritt im Bereich der Bodenwand 49 aus der Speicherkammer aus und endet in geringem Abstand vor dem Luftfilter 3e in der an die Innenseite des Luftfilters 3e angrenzenden Kammer 4e.

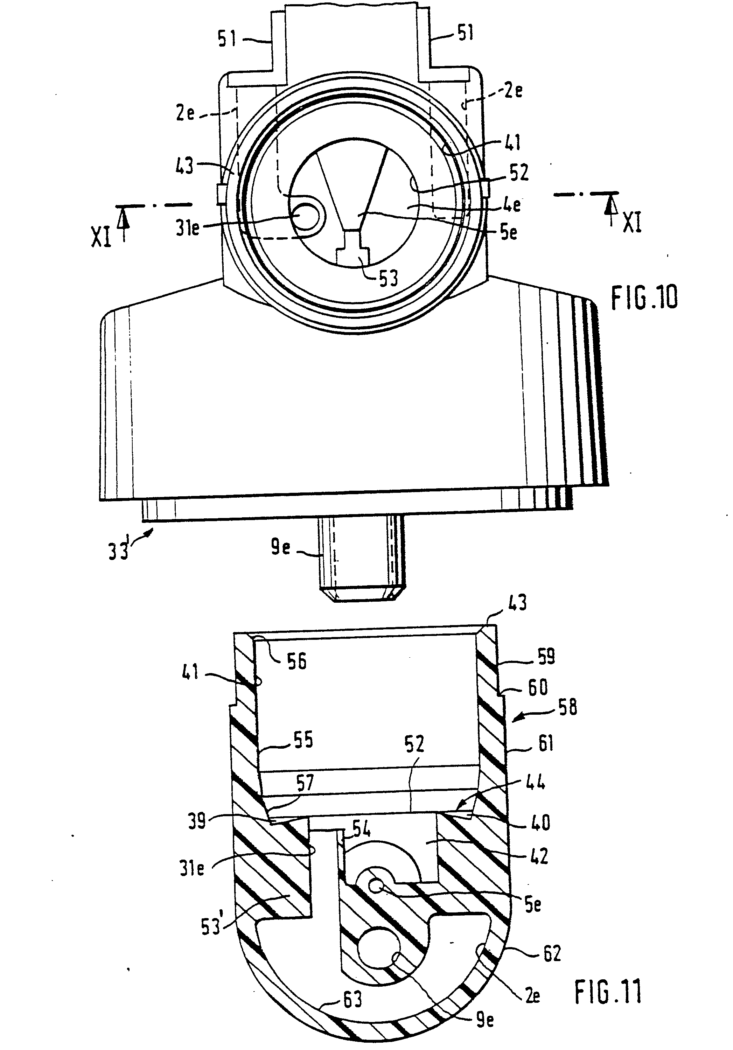

Fig. 10 zeigt eine vergrößerte Seitenansicht der Vorrichtung 1e gemäß Fig. 9 aus Richtung des Pfeiles X, wobei aus Gründen der Übersichtlichkeit das Halteteil 34 und das Luftfilter 3e nicht eingezeichnet sind.

Der im Beispielsfalle als Zylinder ausgebildete Raum 41 weist an seinem in der Vorrichtung 1e liegenden Ende eine Querschnittsverengung auf, wodurch eine im Beispielsfalle kreisrunde Ausnehmung 52 gebildet wird, vor der das Luftfilter 3e angeordnet und mittels des Halteteils 34 gemäß Fig. 9 festgelegt wird. Die Ausnehmung 52 verbindet den Raum 41 mit der an die Innenseite des Luftfilters angrenzenden Kammer 4e, in die die Luftleitung 5e an der mit 53 bezeichneten Stelle mündet. Die in der Darstellung gemäß Fig. 10 nicht sichtbare Speicherkammer 2e ist strichliert angedeutet, wobei der mit der Kammer 2e in Verbindung stehende Luftkanal 31 in der gewählten Darstellung links von der Luftleitung 5e mündet. Ebenso ist es möglich, daß der Luftkanal 31e rechts von der Luftleitung 5e in die Kammer 4e eintritt, wobei bei beiden Möglichkeiten der Luftkanal 31e weder mit der Luftleitung 5e noch mit der Flüssigkeitsleitung 9e in Verbindung steht.

In der Schnittdarstellung gemäß Fig. 11, in der ebenfalls das Halteteil 34 und das Luftfilter 3e aus Gründen der Übersichtlichkeit nicht eingezeichnet sind, ist die Ausbildung der Speicherkammer 2e als Ringraum verdeutlicht, der sich in einem Winkel von 180° im Beispielsfalle um die Flüssigkeitsleitung 9e herum erstreckt. Der die Kammer 4e und die Speicherkammer 2e verbindende Luftkanal 31e ist im Beispielsfalle von einer äußeren Gehäusewandung 53' und einem in der gewählten Darstellung innen liegenden Steg 54 begrenzt, so daß er weder mit dem Luftkanal 5e noch mit dem Flüssigkeitskanal 9e in Verbindung steht. Die in der Zeichnung als Spaltbereiche 39 u. 40 dargestellten Ausnehmungen bilden aufgrund der Kreisform des Raumes 41 einen durchgehenden ringförmigen Spaltraum 44, in den sich das vor der Ausnehmung 52 gemäß Fig. 9 angeordnete Luftfilter 3e in der beschriebenen Weise ausdehnt.

Der Raum 41 weist eine zylindrische Innenfläche 55 auf, die in eine an die Stirnfläche 43 angrenzende kegelstumpfförmige Fläche 56 übergeht und mit einer nach innen gerichtet kegelstumpfförmig ausgebildeten Fläche 57 an den Spaltraum 44 angrenzt. Eine äußere Wandfläche 58 weist einen Wandbereich 59 auf, der sich von der Stirnfläche 43 bis zu einem Absatz 60 erstreckt, von dem ab sich ein Wandbereich 61 mit etwas größerem Druchmesser als der Wandbereich 59 bis zu einem halbkreisförmigen Wandbereich 62 erstreckt, der parallel zu einer inneren Wandfläche 63 der ringförmigen Speicherkammer 2e verläuft.

Die insbesondere als Universalinfusionsgerät geeignete sechste Ausführungsform der erfindungsgemäßen Vorrichtung bietet den Vorteil einer besonders raumsparenden, kompakten Bauform, wobei die insbesondere bei der fünften Ausführungsform gemäß den Fig. 5 bis 8 erläuterte Funktionsweise in gleichem Umfange erfüllt wird. Bezüglich der Bemessungen des Luftkanals 31e und dessen Abstand von dem Luftfilter 3e, der Bemessung der Speicherkammer 2e und der Ausbildung des Luftfilters 3e wird auf die bei der Beschreibung der fünften Ausführungsform gemäß den Fig. 5 bis 8 genannten Werte Bezug genommen.

**Patentansprüche**

1. Vorrichtung zur Entnahme von Flüssigkeiten aus insbesondere steril verschlossenen Gefäßen (8 ; 8a ; 8b ; 8c ; 8d ; 8e), mit einer Flüssigkeitsleitung (9 ; 9a ; 9b ; 9c ; 9d ; 9e) zur Förderung der Flüssigkeit (7 ; 7a ; 7b ; 7c ; 7d ; 7e) aus dem Gefäß, einer Luftleitung (5 ; 5a ; 5b ; 5c ; 5d ; 5e) zur druckausgleichenden Förderung von Umgebungsluft in das Gefäß und einem mit der Luftleitung in Verbindung stehenden hydrophoben Luftfilter (3 ; 3a ; 3b ; 3c ; 3d ; 3e), dadurch gekennzeichnet, daß eine von dem Luftfilter (3 ; 3a ; 3b ; 3c ; 3d ; 3e) getrennt angeordnete Speicherkammer (2 ; 2a ; 2b ; 2c ; 2d ; 2e) vorgesehen ist, die bei in die Luftleitung (5 ; 5a ; 5b ; 5c ; 5d ; 5e) eindringender Flüssigkeit (7 ; 7a ; 7b ; 7c ; 7d ; 7e) ein abgeschlossenes Luftpolster (24 ; 24a ; 24b ; 24c ; 24d ; 24e) aufnimmt.

2. Vorrichtung nach Anspruch 1, dadurch gekennzeichnet, daß die Speicherkammer (2 ; 2a ; 2b ; 2c ; 2d ; 2e) mit der an die Innenseite des Luftfilters (3 ; 3a ; 3b ; 3c ; 3d ; 3e) angrenzenden Kammer (4 ; 4a ; 4b ; 4c ; 4d ; 4e) verbunden ist.

3. Vorrichtung nach Anspruch 2, dadurch gekennzeichnet, daß ein Lufkanal (31 ; 31e) insbesondere im Falle eines aufrecht angeordneten Luftfilters (3d ; 3e) in geringem Abstand vor dem Luftfilter (3d ; 3e) endet.

4. Vorrichtung nach einem der Ansprüche 1 bis 3, dadurch gekennzeichnet, daß ein in dem geschlossenen Gefäß (8 ; 8a ; 8b ; 8c ; 8d ; 8e) herrschender Unterdruck die in dem Luftspeicher (2 ; 2a ; 2b ; 2c ; 2d ; 2e) befindliche Luft zum Luftfilter (3 ; 3a ; 3b ; 3c ; 3d ; 3e) fördert.

5. Vorrichtung nach Anspruch 3 oder 4, dadurch gekennzeichnet, daß der Luftkanal (31 ; 31e) einen geringen Durchmesser von wenigstens annähernd 0,3 und maximal annähernd 3 mm, vorzugsweise im Bereich zwischen 1 und 2 mm aufweist.

6. Vorrichtung nach einem der Ansprüche 1 bis 5, dadurch gekennzeichnet, daß das Luftfilter (3 ; 3a ; 3b ; 3c ; 3d ; 3e) als hydrophobe Membran mit einer Porengröße von wenigstens annähernd 0,1 bis 2 $\mu$m vorzugsweise von annähernd 0,5 bis 1,2 $\mu$m ausgebildet ist.

7. Vorrichtung nach einem der Ansprüche 1 bis 6, dadurch gekennzeichnet, daß der Luftspeicher (2 ; 2a ; 2b ; 2c ; 2d ; 2e) als integrales Bauteil der Vorrichtung (1 ; 1a ; 1b ; 1c ; 1d ; 1e) mit einem Volumen von wenigstens annähernd 50 $\mu$l bis maximal annähernd 1 000 $\mu$l, vorzugsweise annähernd 100 bis 200 $\mu$l ausgebildet ist.

8. Vorrichtung nach einem der Ansprüche 3 bis 7, dadurch gekennzeichnet, daß der Luftkanal (31 ; 31e) in einem Abstand von wenigstens annähernd 0,1 bis maximal annähernd 2 mm, vorzugsweise annähernd 0,3 bis 0,8 mm vor dem Luftfilter (3d ; 3e) endet.

9. Vorrichtung nach einem der Ansprüche 1 bis 8, dadurch gekennzeichnet, daß der Luftfilter (3a) teilweise luftdicht abgedichtet ist.

10. Vorrichtung nach einem der Ansprüche 1 bis 9, dadurch gekennzeichnet, daß der den Luftfilter (3 ; 3a ; 3b ; 3c) mit dem Luftkanal (5 ; 5a ; 5b ; 5c) verbindende Luftraum (4 ; 4a ; 4b ; 4c) mit einem zusätzlichen kuppelförmigen Sammelraum versehen ist.

11. Vorrichtung nach einem der Ansprüche 1 bis 10, dadurch gekennzeichnet, daß das Luftfilter (3e) mittels einer Schutzeinrichtung (Halteteil 34) vor Beschädigung von außen geschützt ist.

12. Vorrichtung nach einem der Ansprüche 1 bis 11, dadurch gekennzeichnet, daß die Speicherkammer (2e) mittels eines separaten Deckels (51) verschließbar ist.

13. Vorrichtung nach einem der Ansprüche 1 bis 12, dadurch gekennzeichnet, daß das Luftfilter (3e) mittels einer Klemmverbindung (Spaltraum 44) luftdicht abdichtbar ist und in seiner Lage sicherbar ist.

14. Vorrichtung nach einem der Ansprüche 1 bis 13, gekennzeichnet durch ihre Anwendung als Universalinfusionsgerät.

**Claims**

1. An apparatus for running off liquids from in particularly sterile sealed vessels (8 ; 8a ; 8b ; 8c ; 8d ; 8e) comprising a liquid duct (9 ; 9a ; 9b ; 9c ; 9d ; 9e) for discharging said liquid (7 ; 7a ; 7b ; 7c ; 7d ; 7e) from said vessel, an air duct (5 ; 5a ; 5b ; 5c ; 5d ; 5e) for pressure-equalizing introduction of ambient air into said vessel and a hydrophobic air filter (3 ; 3a ; 3b ; 3c ; 3d ; 3e) communicating with said air duct, characterized in that a storage chamber (2 ; 2a ; 2b ; 2c ; 2d ; 2e) being separately arranged from said air filter (3 ; 3a ; 3b ; 3c ; 3d ; 3e) is provided which takes up a closed air cushion (24 ; 24a ; 24b ; 24c ; 24d ; 24e) when liquid (7 ; 7a ; 7b ; 7c ; 7d ; 7e) penetrates into said air duct.

2. Apparatus according to claim 1, characterized in that said storage chamber (2 ; 2a ; 2b ; 2c ; 2d ; 2e) is connected to the chamber (4 ; 4a ; 4b ; 4c ; 4d ; 4e) adjacent to the inner side of said air filter (3 ; 3a ; 3b ; 3c ; 3d ; 3e).

3. Apparatus according to claim 2, characterized in that an air channel (31 ; 31e) ends at small distance before said air filter (3d ; 3e) in particular in case of an upright air filter (3d ; 3e).

4. Apparatus according to one of claims 1 to 3, characterized in that a low pressure existing in the sealed vessel (8 ; 8a ; 8b ; 8c ; 8d ; 8e) conveys the air from said air storage chamber (2 ; 2a ; 2b ; 2c ; 2d ; 2e) to said air filter (3 ; 3a ; 3b ; 3c ; 3d ; 3e).

5. Apparatus according to claim 3 or 4, characterized in that said air channel (31 ; 31e) comprises a small diameter of at least about 0.3 and maximum about 3 mm, preferably within a range of between 1 and 2 mm.

6. Apparatus according to one of claims 1 to 5, characterized in that said air filter (3 ; 3a ; 3 ; 3c ; 3d ; 3e) is embodied as hydrophobic diaphragm having a pore size of at least about 0.1 to 2 $\mu$m, preferably of about 0.5 to 1.2 $\mu$m.

7. Apparatus according to one of claims 1 to 6, characterized in that said air storage chamber (2 ; 2a ; 2b ; 2c ; 2d ; 2e) is embodied as integral part of said apparatus (1 ; 1a ; 1b ; 1c ; 1d ; 1e), said air storage chamber having a volume of at least about 50 $\mu$l to maximum about 1 000 $\mu$l, preferably about 100 to 200 $\mu$l.

8. Apparatus according to one of claims 3 to 7, characterized in that said air channel (31 ; 31e) ends at a space of at least about 0.1 to maximum about 2 mm, preferably about 0.3 to 0.8 mm before said air filter (3d ; 3e).

9. Apparatus according to one of claims 1 to 8, characterized in that said air filter (3a) is partly air-tightly sealed.

10. Apparatus according to one of claims 1 to 9, characterized in that said air chamber (4 ; 4a ; 4b ; 4c) connecting said air filter (3 ; 3a ; 3b ; 3c) with said air channel (5 ; 5a ; 5b ; 5c) is provided with an additional dome-like space.

11. Apparatus according to one of claims 1 to 10, characterized in that said air filter (3e) is protected against damages by means of a guard means (keeper part 34).

12. Apparatus according to one of claims 1 to

7

11, characterized in that said storage chamber (2e) is closable by means of a separate cover (51).

13. Apparatus according to one of claims 1 to 12, characterized in that said air filter (3e) is airtightly sealable by means of a clamping device (gap space 44) and can be secured in its position.

14. Apparatus according to one of claims 1 to 13, characterized by its use as universal infusion apparatus.

## Revendications

1. Dispositif de soutirage de fluides de réservoirs (8 ; 8a ; 8b ; 8c ; 8d ; 8e) fermés en particulier de manière stérile, comprenant un conduit à fluide (9 ; 9a ; 9b ; 9c ; 9d ; 9e) pour prélever le fluide (7 ; 7a ; 7b ; 7c ; 7d ; 7e) du réservoir, un conduit à air (5 ; 5a ; 5b ; 5c ; 5d ; 5e) pour introduire de l'air ambiant dans le réservoir, avec équilibrage de pression, ainsi qu'un filtre à air hydrophobe (3 ; 3a ; 3b ; 3c ; 3d ; 3e) communiquant avec le conduit à air, caractérisé par le fait qu'il est prévu une chambre d'accumulation (2 ; 2a ; 2b ; 2c ; 2d ; 2e) qui est disposée séparément du filtre à air (3 ; 3a ; 3b ; 3c ; 3d ; 3e), et qui reçoit un coussin d'air confiné (24 ; 24a ; 24b ; 24c ; 24d ; 24e) lorsque du fluide (7 ; 7a ; 7b ; 7c ; 7d ; 7e) pénètre dans le conduit à air (5 ; 5a ; 5b ; 5c ; 5d ; 5e).

2. Dispositif selon la revendication 1, caractérisé par le fait que la chambre d'accumulation (2 ; 2a ; 2b ; 2c ; 2d ; 2e) est reliée à la chambre (4 ; 4a ; 4b ; 4c ; 4d ; 4e) contiguë à la face interne du filtre à air (3 ; 3a ; 3b ; 3c ; 3d ; 3e).

3. Dispositif selon la revendication 2, caractérisé par le fait que, notamment dans le cas d'un filtre à air (3d ; 3e) disposé verticalement, un canal d'air (31 ; 31e) s'achève à faible distance avant ce filtre à air (3d ; 3e).

4. Dispositif selon l'une des revendications 1 à 3, caractérisé par le fait qu'une dépression régnant dans le réservoir fermé (8 ; 8a ; 8b ; 8c ; 8d ; 8e) achemine, vers le filtre à air (3 ; 3a ; 3b ; 3c ; 3d ; 3e), l'air situé dans l'accumulateur d'air (2 ; 2a ; 2b ; 2c ; 2d ; 2e).

5. Dispositif selon la revendication 3 ou 4, caractérisé par le fait que le canal d'air (31 ; 31e) présente un faible diamètre mesurant au minimum approximativement 0,3 et au maximum approximativement 3 mm, de préférence compris entre 1 et 2 mm.

6. Dispositif selon l'une des revendications 1 à 5, caractérisé par le fait que le filtre à air (3 ; 3a ; 3b ; 3c ; 3d ; 3e) est réalisé sous la forme d'une membrane hydrophobe ayant une grosseur de pores d'au moins approximativement 0,1 à 2 $\mu$m, de préférence d'approximativement 0,5 à 1,2 $\mu$m.

7. Dispositif selon l'une des revendications 1 à 6, caractérisé par le fait que l'accumulateur d'air (2 ; 2a ; 2b ; 2c ; 2d ; 2e) est réalisé en tant que partie constitutive intégrante du dispositif (1 ; 1a ; 1b ; 1c ; 1d ; 1e), ayant un volume d'au moins approximativement 50 $\mu$l à approximativement 1 000 $\mu$l au maximum, de préférence compris entre approximativement 100 et 200 $\mu$l.

8. Dispositif selon l'une des revendications 3 à 7, caractérisé par le fait que le canal d'air (31 ; 31e) s'achève, avant le filtre à air (3d ; 3e), à une distance d'au moins approximativement 0,1 à approximativement 2 mm au maximum, de préférence comprise entre approximativement 0,3 et 0,8 mm.

9. Dispositif selon l'une des revendications 1 à 8, caractérisé par le fait que le filtre à air (3a) est obturé en partie avec étanchéité à l'air.

10. Dispositif selon l'une des revendications 1 à 9, caractérisé par le fait que la chambre à air (4 ; 4a ; 4b ; 4c) reliant le filtre à air (3 ; 3a ; 3b ; 3c) au canal d'air (5 ; 5a ; 5b ; 5c) est pourvue d'une chambre collectrice supplémentaire, en forme de dôme.

11. Dispositif selon l'une des revendications 1 à 10, caractérisé par le fait que le filtre à air (3e) est protégé de détériorations, de l'extérieur, au moyen d'un dispositif protecteur (partie de retenue 34).

12. Dispositif selon l'une des revendications 1 à 11, caractérisé par le fait que la chambre d'accumulation (2e) peut être obturée au moyen d'un couvercle distinct (51).

13. Dispositif selon l'une des revendications 1 à 12, caractérisé par le fait que le filtre à air (3e) peut, au moyen d'une liaison par coincement (intervalle de séparation 44), être obturé avec étanchéité à l'air et être verrouillé à demeure.

14. Dispositif selon l'une des revendications 1 à 13, caractérisé par son utilisation en tant qu'appareil perfuseur universel.

FIG.1

FIG. 2

FIG.3

FIG.4

1

0 085 957

FIG.5

FIG.6

FIG.7

FIG.8

2

FIG.9

FIG.10

FIG.11

4